# EUROPÄISCHE PATENTSCHRIFT

(11) **EP 1 231 906 B1**
(45) Veröffentlichungstag und Bekanntmachung des Hinweises auf die Patenterteilung: **21.02.2007**
(21) Anmeldenummer: 00976023.2
(22) Anmeldetag: 11.11.2000
(51) Int. Cl.: A61K 9/70

(54) **TRANSDERMALES THERAPEUTISCHES SYSTEM ENTHALTEND LEICH FLÜCHTIGE WIRKSTOFFE**
TRANSDERMAL THERAPEUTIC SYSTEM CONTAINING HIGHLY VOLATILE ACTIVE INGREDIENTS
SYSTEME THERAPEUTIQUE TRANSDERMIQUE CONTENANT DES PRINCIPES ACTIFS TRES VOLATILES

(30) Priorität: 27.11.1999 DE 19957234
(43) Veröffentlichungstag der Anmeldung: 21.08.2002
(73) Patentinhaber: HEXAL AG, 83607 Holzkirchen (DE)
(72) Erfinder: KIBELE, Ralf, Hexal AG, 83607 Holzkirchen (DE); KLOKKERS, Karin, Hexal AG, 83607 Holzkirchen (DE); SCHLÜTER, Hans-Jürgen, Hexal AG, 83607 Holzkirchen (DE)
(86) Internationale Anmeldenummer: PCT/EP2000/011169
(87) Internationale Veröffentlichungsnummer: WO 2001/039752

(56) Entgegenhaltungen:
- WO-A-93/00115
- DE-A- 4 433 191

## Beschreibung

Die Erfindung betrifft ein pharmazeutisches, therapeutisches System in Form eines Pflasters, welches leicht flüchtige Wirkstoffe als aktive Bestandteile in einem durch eine Membran begrenzten Reservoirsystem enthält, wobei die Deckschicht einen reversiblen Verbund, der durch eine Siegelung von annähernd wirkstoffundurchlässigem Polyester gegen eine wirkstoffdurchlässige Polymermembran hergestellt wird, darstellt. Die annähernd wirkstoffundurchlässige Polyesterfolie kann vor Gebrauch abgezogen werden.

Gemäß dem Stand der Technik existiert eine Vielzahl an Patentanmeldungen, die Pflaster beschreiben, welche flüchtige Wirkstoffe, wie z.B. ätherische Öle oder Repellents, enthalten. WO-A-93 00115 offenbart ein Laminat zur controllierten Abgabe von Medikamenten und Repellents. WO 98/17262 beschreibt ein Pflaster, enthaltend ätherische Öle, zur Aromatherapiebehandlung. Die Aufnahme der ätherischen Öle erfolgt ausschließlich transdermal, so daß eine systemische Wirkung die Folge ist. Es wird explizit darauf hingewiesen, daß eine für die flüchtigen Wirkstoffe undurchlässige Deckschicht verwendet wird, um den Verlust an ätherischen Ölen durch Evaporation zu verhindern.
DE 295 11 982 U 1 beschreibt ein Pflaster mit einem Repellent-Duftdepot. Es wird zur Insektenabwehr verwendet. Das Depot wird durch ein mit ätherischen Ölen getränktes Gewebe, Vlies oder Watte gebildet. Als abziehbare Folie wird eine Aluminiumfolie verwendet. Der Nachteil von Aluminiumfolien ist die teure Anschaffung, die bei der Herstellung benötigte große Menge an Energie und die Entsorgung.
DE 197 12 359 A1 beschreibt ein Pflaster, das an der Kleidung oder am Körper angebracht werden kann und ätherische Öle als Wirkstoffe enthält. Es besitzt eine Isolierfolie über der Kleberschicht, um eine transdermale Applikation zu vermeiden. Nur die Evaporation ist von Bedeutung. Das ätherische Öle enthaltende Depot (Wirkstoffreservoir) wird von einer Trägerschicht abgedeckt. Diese Trägerschicht ist wirkstoffdurchlässig und besteht aus einem Vlies aus Polyester, Polyamid, Viskose, Baumwolle und/ oder Zellstoff. Der Nachteil dieses Systems ist, daß bei der Lagerung ätherische Öle aus dem Wirkstoffreservoir durch die wirkstoffdurchlässige Trägerschicht in den Verpackungsraum entweichen. Diese ätherischen Öle lagern sich in der Klebeschicht ein. Das hat zur Folge, daß die Klebkraft des Systems nicht mehr ausreichend ist und eine Auflösung des Klebers von statten geht. Außerdem nimmt der Gehalt an ätherischen Ölen durch den Abdampfvorgang während der Lagerung ganz erheblich ab.
DE 32 16 609 A1 beschreibt ein Pflaster-Inhalat zum Aufbringen von ätherischen Ölen in der Brustregion. Die Wirkstoffe werden perkutan und inhalativ zur Wirkung gebracht. Das Trägersystem ist in einer wirkstoffdurchlässigen Folie (perforiert) eingeschweißt, wobei auf der einen Seite noch eine Klebeschicht aufgebracht ist. Dieses System ist zur Lagerung in kunststoffkaschierten Aluminiumfolien eingesiegelt. Man hat hier wieder das Problem, daß während der Lagerung die entweichenden ätherischen Öle in der Kleberschicht angereichert werden, und somit die Klebkraft auf ein Minimum sinkt. Außerdem nimmt der Gehalt an ätherischen Ölen durch den Abdampfvorgang während der Lagerung ganz erheblich ab.

Die Aufgabe der Erfindung ist es, ein für die Inhalation von leicht flüchtigen Stoffen geeignetes Membranpflaster bereitzustellen, das lagerstabil ist. Lagerstabil bedeutet, daß sich die Konsistenz des Pflasters, welches zur Lagerung z.B. in einer aluminiumhaltigen Verpackung eingeschweißt ist, auch über einen längeren Zeitraum nicht wesentlich ändert, die Klebkraft erhalten bleibt und die Wirkstoffkonzentration nicht wesentlich abnimmt. Die Herstellung sollte gegenüber dem herkömmlichen Membranpflasterherstellungsprozeß keinen zusätzlichen Herstellungsschritt benötigen sowie einfach und preiswert sein. Auf die Umweltverträglichkeit muß ebenfalls geachtet werden.

Die erfindungsgemäße Aufgabe konnte nun überraschend durch die Verwendung des pharmazeutischen, therapeutischen Systems wie im vorliegenden Anspruch 1 gelöst werden.

Das verwendete Polyester kann eine Folie sein, die aus einer oder mehreren Polyesterlagen besteht. Weiter können die Polyesterlage(n) metallisiert oder mit einer Aluminiumschicht kaschiert sein. Unter annähernd wirkstoffundurchlässig wird in diesem Fall eine Permeabilität der Polyesterfolie für leicht flüchtige Wirkstoffe verstanden, die in einem Bereich von 2 x 10⁻⁶ bis 60000 x 10⁻⁶ g/m² x d bei 20°C und einer Dicke von 25 µm liegt.

Unter dem erfindungsgemäßen reversiblen Verbund versteht man einen Verbund aus einer annähernd wirkstoffundurchlässigen Polyesterfolie und einer wirkstoffdurchlässigen Polymermembran, bei der man gegebenenfalls nach dem Aufkleben auf die Haut, die Polyesterfolie leicht von der wirkstoffdurchlässigen Polymermembran abziehen kann, so daß die, eine inhalative Aufnahme gewährleistende, Wirkstofffreisetzung durch die wirkstoffdurchlässige Polymermembran ungestört stattfinden kann. Bei der Lagerung kann durch die annähernd wirkstoffundurchlässige Polyesterschicht praktisch kein Wirkstoff entweichen. Eine Verringerung der Klebkraft durch den während der Lagerung freigesetzten Wirkstoff und eine damit verbundene Konsistenzveränderung des Pflasters kann somit vermieden werden. Der Herstellungsprozeß kann trotzdem wie bei herkömmlichen Pflastern erfolgen, ohne daß ein zusätzlicher Schritt benötigt wird. Die Polyesterfolie wird direkt mit der wirkstoffdurchlässigen Polymermembran, wie z.B. Polyethylen oder Polypropylen, in der Produktionsmaschine gesiegelt. Die Siegelung erfolgt in einem Temperaturbereich von 90 °C - 220 °C, vorzugsweise 140 °C - 190 °C. Die verwendeten Folien sind preiswert in der Anschaffung und umweltgerecht zu entsorgen..

Das erfindungsgemäße pharmazeutische, therapeutische System stellt ein Pflaster dar, welches leicht flüchtige Wirkstoffe als aktive Bestandteile in einem Membransystem enthält, wobei die Deckschicht ein reversiblen Verbund ist, der durch eine Siegelung von annähernd wirkstoffundurchlässigem Polyester gegen eine wirkstoffdurchlässige Polymermembran hergestellt wird.

Als annähernd wirkstoffundurchlässige Polyesterfolie können eine oder mehrere Polyesterlagen, metallisiertes Polyester, Polyesterverbundfolien oder mit Aluminium kaschiertes Polyester verwendet werden.

Das erfindungsgemäße pharmazeutische, therapeutische System kann Polyethylen, Polypropylen, Ethylen-Vinylacetat, Polyurethan, Polyethylenterephthalat (Hytrel^{®}) und/ oder Polyethylen/ Ethylen-Vinylacetat- Copolymere, insbesondere Polyethylen oder Polypropylen, als wirkstoffdurchlässiges Polymer der Deckschicht enthalten.

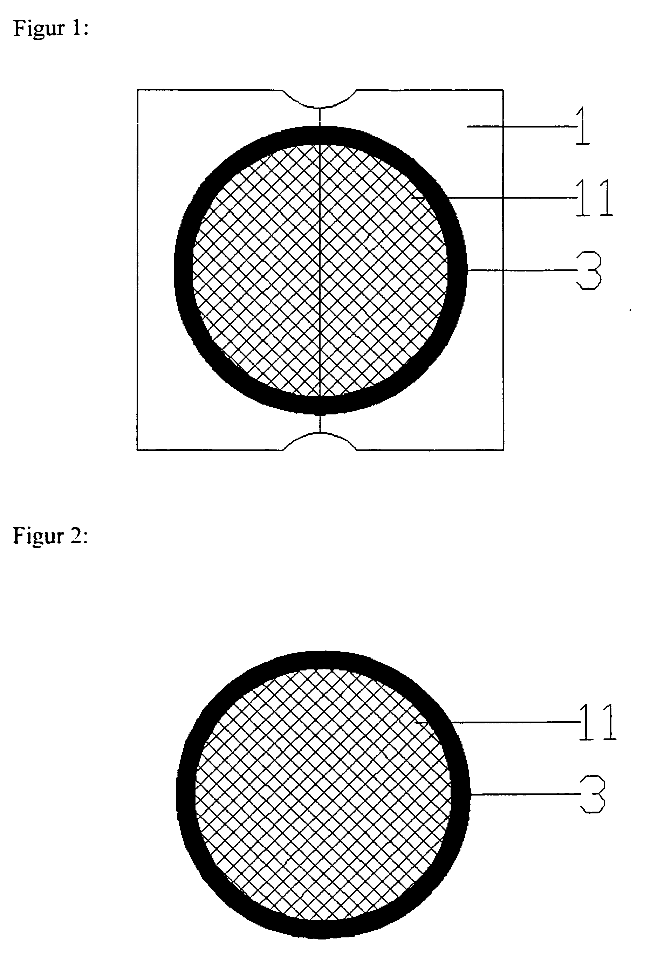

Bei diesem pharmazeutischen, therapeutischen System der Erfindung handelt es sich um ein Membransystem (siehe Figuren 1-9), welches aus einem reversiblen Verbund (11) einer annähernd wirkstoffundurchlässigen Polyesterfolie (9) mit einer wirkstoffdurchlässigen Polymermembran (5) als Deckschicht, einem die leicht flüchtigen Wirkstoffe enthaltenden Reservoir oder einer Reservoirschicht (2), einer für die leicht flüchtigen Wirkstoffe undurchlässigen Dichtfolie (6), einer Haftklebeschicht (7) oder Haftklebering (8) und einer abziehbaren Schutzschicht (1) besteht.

Das Reservoir liegt bei einer möglichen Ausführungsform zwischen einer Membran auf der Oberseite und einer Dichtfolie auf der Unterseite. Die Dichtfolie verhindert eine Permeation der aktiven Bestandteile durch die Haut. Hautirritationen der Reservoirbestandteile können somit auch bei sehr empfindlicher Haut verhindert werden. Die Membran an der Oberseite (ein Teil der erfindungsgemäßen Deckschicht) erlaubt nach Entfernung der annähernd wirkstoffundurchlässigen Polyesterfolie die Freisetzung der leicht flüchtigen Wirkstoffe zur inhalativen Aufnahme durch den Patienten; d.h. die leicht flüchtigen Wirkstoffe gelangen aufgrund ihres Dampfdruckes nach Passieren der Membran durch Inhalation über den Respirationstrakt in die Lunge und entfalten dort ihre lokale Wirkung.

Die annähernd wirkstoffundurchlässige Polyesterfolie verhindert ein Abdampfen der leicht flüchtigen Wirkstoffe und garantiert eine Erhaltung der Klebkraft und der gewünschten Konsistenz während der Lagerung.

Das Reservoir enthält leicht flüchtige Wirkstoffe als aktive Bestandteile, gegebenenfalls Stabilisatoren, Emulgatoren, Verdickungsmittel und/ oder übliche Membransystem- bzw. Reservoirpflaster- Hilfsmittel. Als Stabilisatoren, Emulgatoren und Verdickungsmittel werden die dem Fachmann bekannten Stoffe verwendet. Die bevorzugt verwendeten Verdickungsmittel sind Vaseline, Ölsäureoleylester (Cetiol^{®}), Komplexemulgatorgele (Lanettewachs ASS, Lanette^{®} N), halbsynthetische Fette (Softisan), hochdisperses Siliciumdioxid (Aerosil^{®} und/ oder Bentonit (Veegum^{®}, Volclay^{®}, Ben-A-Gel). Als Gelbildner und damit ebenfalls Verdickungsmittel können bei Bedarf Cellulosederivate, wie z.B. Methylcellulose (Methocel^{®}, Tylose^{®} MH/ Tylose^{®} MB), Hydroxypropylcellulose (Klucel), Hydroxyethylcellulose (Ethoxose), Hydroxypropylmethylcellulose (Methocel^{®} E, Methocel^{®} K) und/ oder Ethylcellulose, sowie Polyacrylsäure (Carbopol^{®}), Carboxyvinylpolymer, Carbomer-Copolymer, Natrium- Plyoxilat, Carboxymethylcellulose oder ein Gemisch aus diesen verwendet werden. Das Reservoir kann aber auch ein Vlies, Gewebe, Papier oder Watte als Träger der aktiven Bestandteile enthalten.

Das erfindungsgemäße pharmazeutische, therapeutische System kann als leicht flüchtige Wirkstoffe ätherische Öle, wie z.B. Menthol, Thymol, Campher, Limonen, Borneol, Camphen, α-Pinen, β-Pinen, Bornylacetat, α-Phellandren, β-Phellandren, Cardinen, Δ³-Caren, α-Silvestren, Eukalyptusöl, Kiefernadelöl, Kiefernöl (Latschenkiefernöl), Terpentinöl, Muskatöl, Fichtennadelöl und/ oder Zedemholzöl, volatile Aromatika, wie z.B. Cineol, Pyrethrine, wie z.B. Pyrethrin I und/ oder Pyrethrin II, und/ oder Pyrethroide, wie z.B. Allethrin I, Teramethrin, Permethrin, Decamethrin und/ oder Fenvalerat, und/ oder Diethyltoluamid und/ oder 2-(2-Hydroxyethyl)-1-piperidin-carbonsäure-methyl-propylester im Reservoir enthalten.

Eine bevorzugte Ausführungsform des erfindungsgemäßen pharmazeutischen, therapeutischen Systems beinhaltet ätherische Öle, insbesondere Kiefernadelöl, Eukalyptusöl und gegebenenfalls Campher und/ oder Menthol als leicht flüchtige Wirkstoffe im Reservoir.

Der Gehalt an Eukalyptusöl kann dabei 10-60 Prozent, insbesondere 15-40 Prozent, bezogen auf die Gesamtmasse der Reservoirbefüllung, betragen.

Der Gehalt an Kiefernadelöl bzw. Latschenkiefernöl bzw. Kiefernöl kann dabei 10-60 Prozent, insbesondere 15-40 Prozent, bezogen auf die Gesamtmasse der Reservoirbefüllung, betragen.

Der Gehalt des gegebenenfalls zugefügten Camphers kann dabei 1-30 Prozent, bezogen auf die Gesamtmasse der Reservoirbefüllung, betragen.

Der Gehalt des gegebenenfalls zugefügten Menthols kann dabei 1-35 Prozent, bezogen auf die Gesamtmasse der Reservoirbefüllung, betragen.

Der Gehalt des gegebenenfalls zugefügten Cineols kann dabei 5-30 Prozent, bezogen auf die Gesamtmasse der Reservoirbefüllung, betragen.

Die besonders gut geeigneten leicht flüchtigen Wirkstoffe sind Kiefernadelöl und/ oder Eukalyptusöl. Der Gehalt an Kiefernadelöl kann 0-80 Gew.% und an Eukalyptusöl 10-100 Gew.%, bezogen auf den Gesamtgehalt an ätherischen Ölen, betragen. Bevorzugt wird ein Gehalt an Kiefernadelöl von 50 Gew.% und Eukalyptusöl von 50 Gew.% oder ein Gehalt an Eukalyptusöl von 100 Gew.%, bezogen auf den Gesamtgehalt an ätherischen Ölen im Reservoir. Der gesamte Gehalt an leicht flüchtigen Wirkstoffen kann 40 - 50 Gew. %, bezogen auf die Menge der Gesamtreservoirbefüllung, betragen.

Die Menge der gesamten Reservoirbefüllung kann 0,1-10 g, insbesondere 0,3-3 g betragen.

Die gegebenenfalls verwendete Dichtfolie kann aus mit Polypropylen, Polyethylen, Polyvinylchlorid oder Surlyn beschichtetem Polyamid, Polyester, Aluminium oder Verbundmaterial, das mindestens eine der Komponenten enthält, bestehen. Bei der bevorzugten Ausführungsform wird ein aluminisierter Polyesterverbund als Dichtfolie verwendet.

Für die Haftklebeschicht bzw. den Haftklebering kann man ein druckempfindliches oder hochschmelzendes (hot-melt) Klebemittel, beispielsweise auf Polyurethanbasis, Polyisobutylenbasis, Polyvinyletherbasis, Siliconbasis, Polyacrylatbasis oder ein Gemisch aus diesen, wählen. Bevorzugt wird ein Klebemittel auf Polyacrylatbasis.

In die Haftklebeschicht kann gegebenenfalls natürliches Vitamin E, synthetisches Vitamin E und/ oder Vitamin E- Derivate als hautpflegende, Hautirritationen verhindernde und die Klebkraft verstärkende Komponente gegeben werden.

Bei dem Klebemittel auf Silkonbasis kann es sich um Silikonkleber handeln, welche auf zwei Hauptbestandteilen basieren: Ein Polymer oder Klebstoff, insbesondere Polysiloxan, und ein die Klebrigkeit erhöhendes Harz. Der Polysiloxankleber ist gewöhnlich mit einem Vernetzer für den Kleber, typischerweise mit einem hochmolekularen Polydiorganosiloxan, und mit dem Harz zubereitet, um über ein angemessenes organisches Lösungsmittel eine dreidimensionale Silikatstruktur zu ergeben. Die Zumischung des Harzes zu Polymer ist der wichtigste Faktor, um die physikalischen Eigenschaften der polysiloxanen Kleber zu ändern; vgl. beispielsweise Sobieski, et al., "Silicone Pressure Sensitive Adhesives", Handbook of Pressure Sensitive Adhesive Technology, 2^{nd} ed., pp. 508-517 (D. Satas, ed.), Van Nostrand Reinhold, New Adhesive Technology, 2^{nd} ed., pp. 508-517 (D. Satas, ed.), Van Nostrand Reinhold, New York (1989).

Ein weiteres Beispiel für ein druckempfindliches Klebemittel auf Silikonbasis ist trimethyliertes Siliciumdioxid, das mit Polydimethylsiloxan mit endständigen Trimethylsiloxy- Gruppen behandelt worden ist.

Bei den Klebemitteln auf Polyacrylatbasis kann es sich um ein beliebiges Homopolymer, Copolymer oder Terpolymer, bestehend aus verschiedenen Acrylsäurederivaten handeln.

So können die Polyacrylate Polymere eines oder mehrerer Monomere von Acrylsäuren und anderen copolymerisierbaren Monomeren sein. Außerdem können die Acrylatpolymere Copolymere von Alkylacrylaten und/ oder -methacrylaten und/ oder copolymerisierbaren sekundären Monomeren oder Monomeren mit funktionellen Gruppen umfassen. Verändert man den Betrag jeder Sorte, die als Monomer hinzugefügt ist, können die kohäsiven Eigenschaften der daraus resultierenden Acrylatpolymere verändert werden. Im allgemeinen besteht das Acrylatpolymer aus mindestens 50 Gew.-% eines Acrylat-, Methacrylat-, Alkylacrylat- oder Alkylmethacrylat-Monomers, 0 bis 20 % eines funktionellen Monomers, copolymerisierbar mit Acrylat, und 0 bis 50 % eines anderen Monomeren.

Im folgenden sind verschiedene Acrylatmonomere, wie z.B. Acrylsäure, Methacrylsäure, Butylacrylat, Butylmethacrylat, Hexylacrylat, Hexylmethacrylat, Isooctylacrylat, Isooctylmethacrylat, Glycidylmethacrylat, 2-Hydroxyethylacrylat, Methylacrylat, Methylmethacrylat, 2-Ethylhexylacrylat, 2-Ethylhexylmethacrylat, Decylacrylat, Decylmethacrylat, Dodecylacrylat, Dodecylmethacrylat, Tridecylacrylat und Tridecylmethacrylat, aufgeführt, die alleine oder in Mischung polymerisiert werden können.

Zusätzlich können funktionelle Monomere, die mit den oben genannten Acrylaten copolymerisierbar sind, wie beispielsweise Acrylsäure, Methacrylsäure, Maleinsäure, Maleinanhydrid, Hydroxyethylacrylat, Hydroxypropylacrylat, Acrylamid, Dimethylacrylamid, Acrylnitril, Dimethylaminoethylacrylat, Dimethylaminoethylmethacrylat, tert.-Butylaminoethylacrylat, ter.-Butylaminoethylmethacrylat, Methoxyethylacrylat, Vinylacetat und Methoxyethylmethacrylat, zur Copolymerisierung eingesetzt werden.

Weiter Einzelheiten und Beispiele für druckempfindliche Acrylate, welche für die Erfindung geeignet sind, sind in Satas Handbook of Pressure Sensitive Adhesive Technology "Acrylic Adhesives", 2^{nd} ed., pp. 396-456 (D. Satas, ed.), Van Nostrand Reinhold, New York (1989) beschrieben.

Für die abziehbare Schutzschicht (Release Liner) kommen Polyester, Polyethylen, Polypropylen, Polysiloxan, Polyacrylat, Ethylenvinylacetat, Polyurethan, Polyisobuten oder Papier, meistens mit Silikon- und /oder Polyethylen beschichtet, oder ein Verbund aus diesen in Betracht. Die bevorzugte Ausführungsform beinhaltet silikonisiertes Papier oder silikonisiertes Polyester als abziehbare Schutzschicht.

Das erfindungsgemäße pharmazeutische, therapeutische System kann eine Fläche von 1 bis 100 cm², insbesondere von 5-50 cm² aufweisen. Die bevorzugten Größen sind 5, 10, 15, 20, 25 oder 30 cm². Das erfindungsgemäße pharmazeutische, therapeutische System kann in verschiedenen Formen vorliegen.

Das erfindungsgemäße therapeutische System kann sowohl für Erwachsene als auch für Babys und Kinder verwendet werden. Die für Kinder und Babys geeignete Ausführungsform beinhaltet keinen Campher und/ oder Menthol, geringere Mengen an Kiefernadel- und/ oder Eukalyptusöl und weist eine Fläche von 5-20 cm², insbesondere 5, 10, 15, 20 cm² auf. Die annähernd wirkstoffundurchlässige Polyesterfolie der Deckschicht kann auf der Außenseite mit verschiedenen Motiven wie Comic-Figuren, Märchengestalten, etc. bedruckt sein.

Nachstehend wird die Erfindung anhand von Figuren noch näher erläutert. Die aufgeführten Figuren zeigen nur den schematischen Aufbau des erfindungsgemäßen Pflasters. Die gezeichneten Schichtdicken entsprechen nicht den wirklichen Schichtdicken. Es zeigen:
**Figur 1**: Draufsicht auf das erfindungsgemäße Pflaster mit einer abziehbaren Schutzschicht (Release Liner) (1), dem reversiblen Verbund (11) als Deckschicht und der Siegelnaht (3)
**Figur 2:** Draufsicht auf ein rundes Pflaster mit einem reversiblen Verbund (11) als Deckschicht und der Siegelnaht (3)

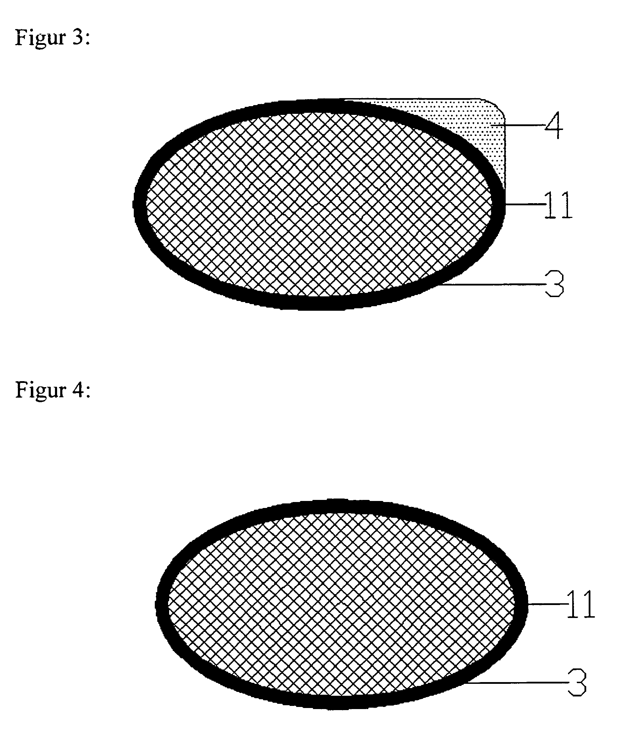

**Figur 3:** Draufsicht auf ein ovales Pflaster mit einem reversiblen Verbund (11) als Deckschicht und der Siegelnaht (3) sowie einem Klebeetikett als Abziehhilfe (4)
**Figur 4**: Draufsicht auf ein ovales Pflaster mit einem reversiblen Verbund (11) als Deckschicht und der Siegelnaht (3)

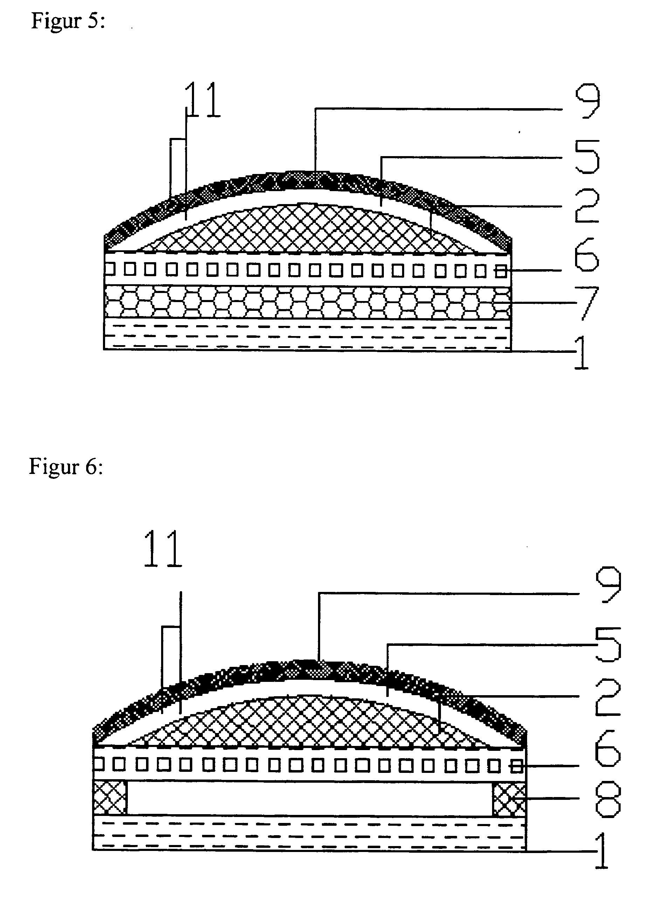

**Figur 5**: Querschnitt durch ein Pflaster mit einer abziehbaren Schutzschicht (1), der Haftklebeschicht (7), der Dichtfolie (6), dem die flüchtigen Wirkstoffe enthaltenden Reservoir (2) und dem erfindungsgemäßen reversiblen Verbund (11), der aus einer für die leicht flüchtigen Wirkstoffe durchlässigen Polymermembran an der Oberseite (5) und einer das Abdampfen der leicht flüchtigen Wirkstoffe während er Lagerung verhindernden Polyesterfolie (9) besteht, als Deckschicht.
**Figur 6**: Querschnitt durch ein Pflaster mit einer abziehbaren Schutzschicht (1), einem Haftklebering (8), der Dichtfolie (6), dem die flüchtigen Wirkstoffe enthaltenden Reservoir (2) und dem erfindungsgemäßen reversiblen Verbund (11), der aus einer für die leicht flüchtigen Wirkstoffe durchlässigen Polymermembran an der Oberseite (5) und einer das Abdampfen der leicht flüchtigen Wirkstoffe während er Lagerung verhindernden Polyesterfolie (9) besteht, als Deckschicht.

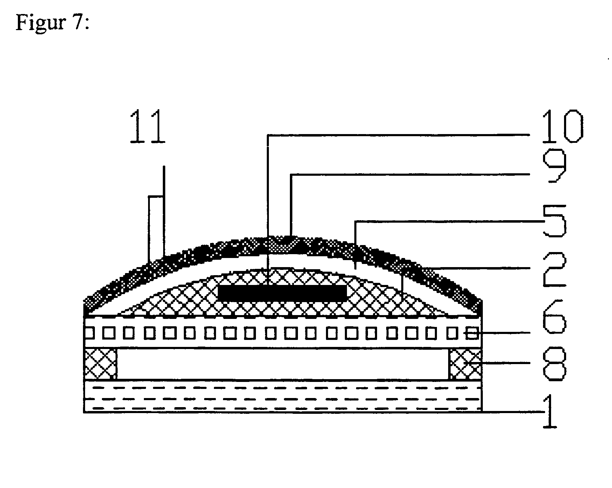

**Figur 7**: Querschnitt durch ein Pflaster mit einer abziehbaren Schutzschicht (1), einem Haftklebering (8), der Dichtfolie (6), dem die leicht flüchtigen Wirkstoffe enthaltenden Reservoir (2), das als Träger der aktiven Bestandteile ein Vlies, Gewebe, Papier oder Watte (10) beinhaltet, und dem erfindungsgemäßen reversiblen Verbund (11), der aus einer für die leicht flüchtigen Wirkstoffe durchlässigen Polymermembran an der Oberseite (5) und einer das Abdampfen der leicht flüchtigen Wirkstoffe während er Lagerung verhindernden Polyesterfolie (9) besteht, als Deckschicht.

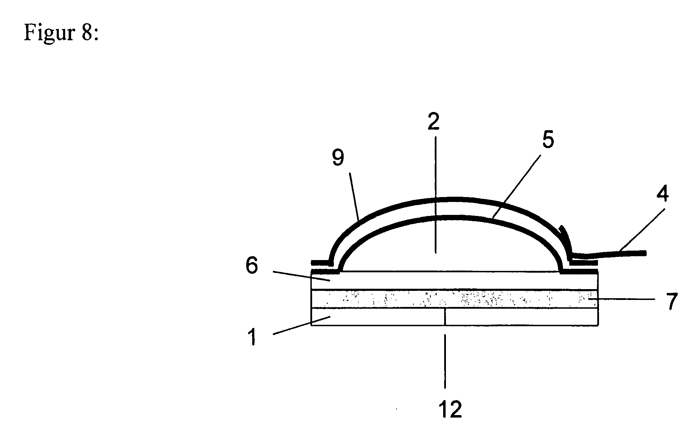

**Figur 8**: Querschnitt durch ein Pflaster mit einer abziehbaren Schutzschicht (1), der Haftklebeschicht (7), der Dichtfolie (6), dem die flüchtigen Wirkstoffe enthaltenden Reservoir (2) und dem erfindungsgemäßen reversiblen Verbund (11), der aus einer für die leicht flüchtigen Wirkstoffe durchlässigen Polymermembran an der Oberseite (5) und einer das Abdampfen der leicht flüchtigen Wirkstoffe während er Lagerung verhindernden Polyesterfolie (9) besteht, als Deckschicht und einem Klebeetikett (4) sowie einer Applizierhilfe (12) in der abziehbaren Schutzschicht.

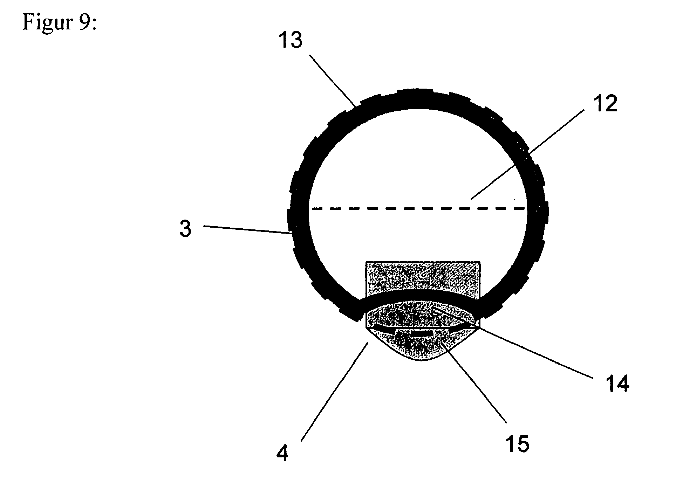

**Figur 9**: Draufsicht auf ein rundes Pflaster mit einem Siegelring (3), das durch eine Stanzung vom übrigen System abgetrennt wurde, und einem Klebeetikett (4), das eine Klebefläche (14) und eine nicht klebende Fläche (15) besitzt. Außerdem ist noch die Stanzung (12) der abziehbaren Schutzschicht (1) zu sehen, die die Funktion einer Applizierhilfe hat.

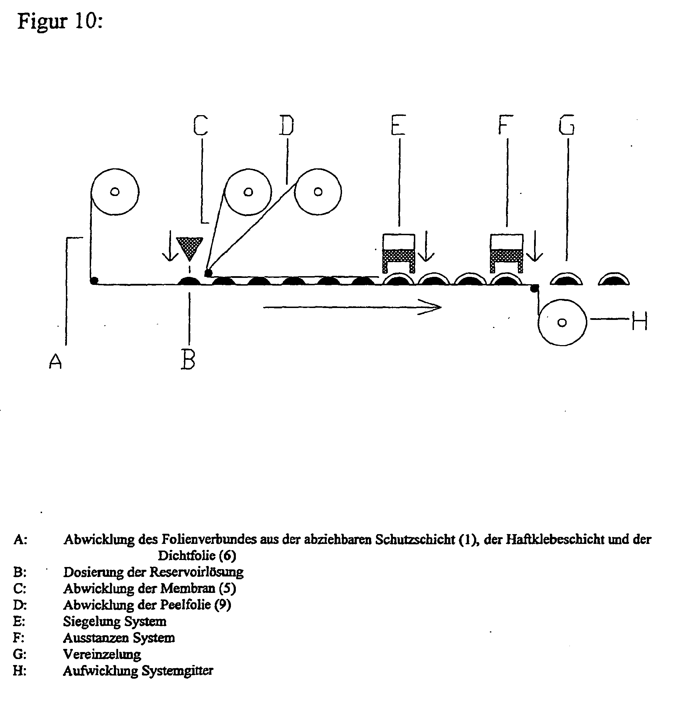

**Figur 10**: Herstellverfahren: Zuerst wird ein Folienverbund aus der abziehbaren Schutzschicht (1), der Haftklebeschicht (7) und der Dichtfolie (6) abgewickelt (A). Dann erfolgt die Dosierung der Reservoirlösung (B). Anschließend wird die wirkstoffdurchlässige Membran (5) (C) und direkt im Anschluß die Peelfolie (9) (annähernd wirkstoffundurchlässige Polyesterfolie) (D) abgewickelt. Als nächster Schritt erfolgt die Siegelung des Sytems (E). Dann werden die Pflaster ausgestanzt (F) und vereinzelt (G). Parallel zur Vereinzelung der Pflaster wird die überschüssige Folie aufgewickelt (H).

Die Erfindung wird zudem durch nachstehende Beispiele näher erläutert.

### Beispiel 1:

Aufbau eines Membransystem-Pflasters zur ausschließlich inhalativen Applikation von ätherischen Ölen mit einer Fläche von 30 cm²:
* abziehbare Schutzschicht: silikonbeschichtetes Papier
* Haftklebeschicht: Polyisobutylen (MA-24) mit 7,5 % Copherol^{®} (Vitamin E) als hautpflegende und die Klebkraft verstärkende Komponente
* für die aktiven Bestandteile undurchlässige Dichtfolie: Verbund aus orientiertem Polyamid 15 µm, Polyethylenterephthalat 12 µm und Polyethylen 30 µm
* Reservoirbefüllung: 1-2 Gew.% Methylcellulose (Tylose^{®} H300), 40-50 Gew.% aktive Bestandteile (Kiefernadelöl : Eukalyptusöl = 1:1) und der Rest Wasser, bei einer Gesamtreservoirbefüllungsmenge von 1,5 g.
* für die aktiven Bestandteile durchlässige Membran, die das die aktiven Bestandteile enthaltende Reservoir vollständig umschließt: Polypropylen (BP Microporous PP Film)
* annähernd wirkstoffundurchlässige Polyesterfolie, die auf die auf der Oberseite des Reservoirs befindlichen wirkstoffdurchlässigen Membran reversibel aufgesiegelt ist.

### Beispiel 2:

Aufbau eines Membransystem-Pflasters zur ausschließlich inhalativen Applikation von ätherischen Ölen mit einer Fläche von 30 cm²:
* abziehbare Schutzschicht: silikonbeschichtetes Papier
* Haftklebeschicht: Polyisobutylen (MA-24) mit 7,5 % Copherol^{®} (Vitamin E) als hautpflegende und die Klebkraft verstärkende Komponente
* für die aktiven Bestandteile undurchlässige Dichtfolie: Verbund aus orientiertem Polyamid 15 µm, Polyethylenterephthalat 12 µm und Polyethylen 30 µm
* Reservoirbefüllung: 1-2 Gew.% Methylcellulose (Tylose^{®} H300), 40-50 Gew.% aktiver Bestandteil (Eukalyptusöl) und der Rest Wasser, bei eine Gesamtreservoirbeftillungsmenge von 1,5 g.
* für die aktiven Bestandteile durchlässige Membran, die das die aktiven Bestandteile enthaltende Reservoir vollständig umschließt: Polypropylen (BP Microporous PP Film)
* annähernd wirkstoffundurchlässige Polyesterfolie, die auf die auf der Oberseite des Reservoirs befindlichen wirkstoffdurchlässigen Membran reversibel aufgesiegelt ist.

### Beispiel 3:

Im ersten Schritte wird die Reservoirfüllung über ein geeignetes Auftragssystem auf einen Verbund, der aus einer abziehbaren Schutzschicht (1), einer Haftklebeschicht (7) und einer Dichtfolie (6) besteht und abgewickelt wird (A), aufgebracht (B). Danach werden sowohl die Membran (5) in einem Abwickelvorgang (C) als auch die Polyesterfolie (9) in einem Abwickelvorgang (D) darübergeführt und das gesamte System wird so gesiegelt, daß eine permanente Verbindung des Verbundes mit der Membran (5) und eine temporäre (peelfähige) Verbindung mit der Polyesterfolie (Peelfolie) (9) entsteht. Als nächstes erfolgt die Stanzung des Einzelsystems (F) über ein geeignetes Schneidwerkzeug (z.B. Stanzzylinder oder Bandstahlschnitte). Die Systeme können vereinzelt (G) und in geeigneter Weise weiter verpackt werden.

Grundsätzlich kann die Aufbringung der Reservoirfüllung horizontal oder vertikal erfolgen, wobei bei der vertikalen Befüllung der Siegelvorgang bereits vor der Befüllung beginnen muß. Auch kann die Siegelung der Dichtfolie (6) auf die Membran (5) in zwei Schritten erfolgen, so daß zuerst nur die Membran (5) und im zweiten Schritt die Dichtfolie (6) gesiegelt wird. Auch können gegebenenfalls mehrere Dichtfolien übereinander gesiegelt werden. Ebenso kann die Stanzung in zwei oder mehreren Schritten erfolgen, so daß zunächst die Form des Pflasters und dann die Außenkontur der Trägerfolie gestanzt wird und die Stanzgitter jeweils separat abgezogen und aufgewickelt (H) werden.

## Patentansprüche

1. Pharmazeutisches, therapeutisches System in Form eines Pflasters, welches leicht flüchtige Wirkstoffe als aktive Bestandteile in einem Membransystem enthält, **gekennzeichnet dadurch, daß** das Membransystem
• einen reversiblen Verbund einer annähernd wirkstoffundurchlässigen Polyesterfolie mit einer wirkstoffdurchlässigen Polymermembran als Deckschicht,
• ein Reservoir oder eine Reservoirschicht, enthaltend die leicht flüchtigen Wirkstoffe,
• eine Dichtfolie,
• eine Haftklebeschicht,
• eine abziehbare Schutzschicht
umfaßt.

2. System gemäß Anspruch 1, **gekennzeichnet dadurch, daß** das annähernd wirkstoffundurchlässige Polyester mit der wirkstoffdurchlässigen Polymermembran durch Siegelung reversibel verbunden ist.

3. System gemäß Anspruch 1 - 2, **gekennzeichnet durch** eine oder mehrere Polyesterlagen, metallisiertes Polyester, Polyesterverbundfolien oder mit Aluminium kaschiertes Polyester als annähernd wirkstoffundurchlässige Polyesterfolie.

4. System gemäß Anspruch 1 - 2, **gekennzeichnet dadurch, daß** die wirkstoffdurchlässige Polymermembran aus Polyethylen, Polypropylen, Ethylen-Vinylacetat, Polyurethan, Polyethylenterephthalat (Hytrel) und/ oder Polyethylen/ Ethylen-Vinylacetat- Copolymere, insbesondere aus Polyethylen oder Polypropylen, besteht.

5. System gemäß Anspruch 1, **gekennzeichnet durch** eine Dichtfolie bestehend aus Polypropylen, Polyethylen, Polyvinylchlorid oder Surlyn^{®} beschichtetes Polyamid, Polyester oder Aluminium sowie Verbundfolien, insbesondere aluminisierte Polyesterverbunde.

6. System gemäß Anspruch 1, **gekennzeichnet durch** eine Haftklebeschicht oder einen Haftklebering bestehend aus druckempfindlichem oder hochschmelzendem Klebemittel auf der Basis von Polyurethan, Polyisobutylen, Polyvinylether, Silicon und/ oder Acrylatbasis, insbesondere ein Klebemittel auf Acrylatbasis.

7. System gemäß Anspruch 6, **gekennzeichnet dadurch, daß** gegebenenfalls natürliches Vitamin E, synthetisches Vitamin E und/ oder Vitamin E- Derivate in der Haftklebeschicht enthalten sind.

8. System gemäß Anspruch 1, **gekennzeichnet durch** eine abziehbare Schutzschicht aus Polyester, Polyethylen, Polypropylen, Polysiloxan, Polyacrylat, Ethylenvinylacetat, Polyurethan, Polyisobuten oder Papier, meistens mit Silikon- und /oder Polyethylen beschichtet, oder ein Verbund aus diesen, insbesondere silikonisiertes Papier oder silikonisiertes Polyester.

9. System gemäß einem der vorangegangenen Ansprüche, **gekennzeichnet durch** ätherische Öle, volatile Aromatika, Pyrethrine, Pyrethroide, Diethyltoluamid und/ oder 2-(2-Hydroxyethyl)-1-piperidin-carbonsäure-methyl-propylester als leicht flüchtige Wirkstoffe.

10. System gemäß Anspruch 9, **dadurch gekennzeichnet, daß** die leicht flüchtigen Wirkstoffe Menthol, Thymol, Campher, Limonen, Bomeol, Camphen, α-Pinen, β-Pinen, Bornylacetat, α-Phellandren, β-Phellandren, Cardinen, Δ³-Caren, α-Silvestren, Eukalyptusöl, Kiefernadelöl, Latschenkiefernöl, Terpentinöl, Muskatöl, Fichtennadelöl, Cineol und/ oder Zedernholzöl sind.

11. System gemäß Anspruch 9 oder 10, **dadurch gekennzeichnet, daß** die leicht flüchtigen Wirkstoffe Kiefernadelöl und/ oder Eukalyptusöl und gegebenenfalls Campher und/ oder Menthol sind.

12. System gemäß Anspruch 9, 10 oder 11, **gekennzeichnet durch** einen Gehalt an Kiefernadelöl von 0-80 Gew.% und an Eukalyptusöl von 10-100 Gew.%, insbesondere ein Gehalt an Kiefernadelöl von 50 Gew.% und Eukalyptusöl von 50 Gew.% oder ein Gehalt an Eukalyptusöl von 100 Gew.%, bezogen auf den Gesamtgehalt an ätherischen Ölen im Reservoir.

13. System gemäß Anspruch 1, **gekennzeichnet durch** gegebenenfalls Vlies, Gewebe, Papier und/ oder Watte als Träger der aktiven Bestandteile und/ oder Stabilisatoren, Emulgatoren, Verdickungsmittel und/ oder andere übliche Hilfsstoffe als weitere Reservoirbestandteile neben den aktiven Bestandteilen.

14. System gemäß Anspruch 1, **gekennzeichnet durch** eine gesamte Reservoirbefüllung von 0,1-10 g, insbesondere 0,3-3 g.

15. System gemäß einem der vorangegangenen Ansprüche, **gekennzeichnet durch** eine Pflasterfläche von 1-100 cm², insbesondere 5-50 cm² und bevorzugt 5, 10, 15, 20, 25 oder 30 cm².

## Claims

1. Pharmaceutical, therapeutic system in the form of a plaster containing highly volatile agents as active ingredients in a membrane system **characterized in that** the membrane system comprises;
- a reversible compound of polyester sheet which is almost impervious to the agent and a polymer membrane which is pervious to the agent as covering layer,
- a reservoir or a reservoir layer containing the highly volatile agents,
- a sealing layer
- an adhesive layer
- a detachable protective layer

2. System according to claim 1, **characterised by** the fact that the polyester which is almost impervious to the agent is reversibly connected with the polymer membrane which is pervious to the agent by means of sealing.

3. System according to claims 1-2, **characterised by** one or more polyester layers, metallised polyester, polyester compound sheets or aluminium-clad polyester as polyester sheet which is almost impervious to the agent.

4. System according to claims 1-2, **characterised by** the polymer membrane which is pervious to the agent consisting of polyethylene, polypropylene, ethylene vinylacetate, polyurethane, polyethylene terephthalate (Hytrel) and/or polyethylene/ethylene vinylacetate copolymer, especially of polyethylene or polypropylene.

5. System according to claim 1, **characterised by** a sealing layer consisting of polypropylene, polyethylene, polyvinylchloride or Surlyn^{®} coated polyamide, polyester or aluminium as well as compound sheets, especially aluminised polyester compounds.

6. System according to claim 1, **characterised by** an adhesive layer or an adhesive ring consisting of pressure-sensitive or high-melting adhesive substance on the basis of polyurethane, polyisobutylene, polyvinyl ether, silicone and/or on acrylate basis, especially an adhesive substance on the basis of acrylate.

7. System according to claim 6, **characterised by** the fact that natural vitamin E, synthetic vitamin E and/or vitamin E derivatives are contained in the adhesive layer if required.

8. System according to claim 1, **characterised by** a detachable protective layer consisting of polyester, polyethylene, polypropylene, polysiloxane, polyacrylate, ethylene vinylacetate polyurethane, polyisobutene or paper, mostly coated with silicone and/or polyethylene, or a compound of these, especially siliconised paper or siliconised polyester.

9. System according to one of the previous claims, **characterised by** essential oils, volatile aromatic substances, pyrethrines, pyrethroids, diethyl toluamide and/or 2-(2-hydroxy ethyl)-1-piperidine-carboxylic acid-methyl propyl ester as highly volatile agents.

10. System according to claim 9, **characterised by** the fact that the highly volatile agents are menthol, thymol, camphor, limonene, borneol, camphene, α-pinene, β-pinene, bornyl acetate, α-phellandrene, β-phellandrene, cardinene, Δ³-carene, α-silvestrene, eucalyptus oil, pine needle oil, dwarf pine needle oil, oil of turpentine, nutmeg oil, spruce needle oil, cineole and/or cedar oil.

11. System according to claim 9 or 10, **characterised by** the fact that the highly volatile agents are pine needle oil and/or eucalyptus oil and camphor and/or menthol if required.

12. System according to claim 9, 10 or 11, **characterised by** a content of pine needle oil of 0-80% by weight and a content of eucalyptus oil of 10-100% by weight, especially a content of pine needle oil of 50% by weight and of eucalyptus oil of 50% by weight or a content of eucalyptus oil of 100% by weight referring to the overall content of essential oils in the reservoir.

13. System according to claim 1, **characterised by** fleece, fabric, paper and/or cotton wool as carrier of the active ingredients and/or stabilisors, emulsifiers, thickening agents and/or other customary adjuvants as further reservoir ingredients in addition to the active ingredients.

14. System according to claim 1, **characterised by** a total reservoir filling of 0.1 - 10 g, especially of 0.3 - 3 g.

15. System according to one of the previous claims, **characterised by** a plaster surface of 1-100 cm², especially of 5-50 cm² and preferably of 5, 10, 15, 20, 25 or 30 cm².

## Revendications

1. Système pharmaceutique et thérapeutique sous forme de pansement, qui renferme des principes actifs légèrement volatiles constituant des éléments actifs d'un système membranaire, **caractérisé par le fait que** le système membranaire comprend :
• Un composite réversible d'un film polyester relativement imperméable aux principes actifs et d'une membrane polymère perméable aux principes actifs constituant une couche protectrice
• Un réservoir ou une « couche-réservoir » contenant les principes actifs légèrement volatiles,
• Une bande d'étanchéité,
• Une feuille adhésive sensible à la pression,
• Une couche protectrice retirable.

2. Système conforme à la revendication 1, **caractérisé par le fait que** le polyester relativement imperméable aux principes actifs soit associé de manière réversible à la membrane polymère perméable aux principes actifs et ce, par un système de fermeture.

3. Système conforme aux revendications 1-2, **caractérisé par** une ou plusieurs couches de polyester, par du polyester métallisé, des films composites, ou du polyester revêtu d'aluminium constituant un film polyester relativement imperméable aux principes actifs.

4. Système conforme aux revendications 1-2, **caractérisé par le fait que** la membrane perméable aux principes actifs se compose de polyéthylène, de polypropylène, d'éthylène-vinylacétate, de polyuréthane, de polyéthylène téréphtalate (Hytrel) et/ ou de polyéthylène/ éthylène-vinylacétate-copolymère, et en particulier de polyéthylène ou de polypropylène.

5. Système conforme à la revendication 1, **caractérisé par** une bande d'étanchéité composée de polypropylène, de polyéthylène, de polyamide revêtu de chlorure de polyvinyle ou de Surlyn^{®}, de polyester ou d'aluminium, ainsi que de film composite, en particulier de composites de polyester aluminisés.

6. Système conforme à la revendication 1, **caractérisé par** une feuille adhésive sensible à la pression ou un anneau adhésif sensible à la pression composé d'un adhésif sensible à la pression ou à point de fusion élevé se basant sur du polyuréthane, du polyisobutylène, de l'éther polyvinylique, du silicone, et/ ou de l'acrylate, en particulier d'adhésif à base d'acrylates.

7. Système conforme à la revendication 6, **caractérisé**, le cas échéant, par le fait que la feuille adhésive sensible à la pression contienne de la vitamine E naturelle, de la vitamine E synthétique et/ ou des dérivés de vitamine E.

8. Système conforme à la revendication 1, **caractérisé par** une couche protectrice retirable composée de polyester, de polyéthylène, de polypropylène, de polysiloxane, de polyacrylate, d'éthylène-vinylacétate, de polyuréthane, de polyisobutène ou de papier, revêtu la plupart du temps d'une couche de silicone et/ ou de polyéthylène, ou d'un composite réversible, constitué en particulier de papier siliconisé ou de polyester siliconisé.

9. Système conforme à une des revendications précédentes, **caractérisé par** des huiles éthériques, de substances aromatiques volatiles, de pyréthrine, de pyréthroide, de diéthyltoluamide, et/ ou de 2-(2-hydroxyéthylène)-1-pipéridine-acides de carbone-méthyl-ester propylique, constituant des principes actifs légèrement volatiles.

10. Système conforme à la revendication 9, **caractérisé par le fait que** le menthol, le thymol, le camphre, le limonène, le bornéol, le camphène, l' α-pinène, le β-pinène, l' acétate de bornyle, l' α-phellandre, le β-phellandre, le cardinène, l' Δ-3-carène, l'α-sylvestrène, l'huile d'eucalyptus, l'huile de résine de pin, l'huile de pin de montagne, l'huile de térébenthine, l'huile de muscat, l'huile de sapin, le cinéole, et/ ou l'huile de bois de cèdre constituent les principes actifs légèrement volatiles.

11. Système conforme aux revendications 9 ou 10, **caractérisé par le fait que** l'huile de résine de pin et/ ou l'huile d'eucalyptus, mais également le camphre et/ ou le menthol constituent les principes actifs légèrement volatiles.

12. Système conforme aux revendications 9, 10 ou 11, **caractérisé par** une teneur en huile de résine de pin de 0-80% en poids et en huile d'eucalyptus de 10-100% du poids, et en particulier une teneur en huile de résine de pin de 50% en poids et d'huile d'eucalyptus de 50% en poids, ou d'une teneur en huile d'eucalyptus de 100% en poids, en référence à la teneur totale en huiles éthériques contenues dans le réservoir.

13. Système conforme à la revendication 1, **caractérisé**, le cas échéant, par une matière non tissée, du tissu, du papier et/ou du coton, porteurs des éléments actifs et/ou des stabilisateurs, des émulsifiants, des gélifiants et/ou d'autres excipients courants constituant d'autres éléments réservoir, en supplément des composants actifs.

14. Système conforme à la revendication 1, **caractérisé par** un remplissage total du réservoir de 0,1-10g, et, en particulier, de 0,3-3g.

15. Système conforme à une des revendications précédentes, **caractérisé par** une zone de pansement de 1-100 cm², en particulier de 5-50 cm², et en priorité de 5, 10, 15, 20, 25 ou 30 cm².
